# EUROPEAN PATENT SPECIFICATION

(11) **EP 1 535 672 B1**
(45) Date of publication and mention of the grant of the patent: **25.01.2012**
(21) Application number: 04380220.6
(22) Date of filing: 05.11.2004
(51) Int. Cl.: B09B 3/00, C05F 1/00, C05F 1/02, C12P 1/04, C12P 1/06, C12P 1/02

(54) **Procedure for eliminating meat wastes especially animal cadavers**
Verfahren zur Beseitigung von Fleischabfällen, insbesondere Tierkadavern
Procédé pour éliminer des déchets de viande, en particulier des cadavres d'animaux

(30) Priority: 25.11.2003 ES 200302751
(43) Date of publication of application: 01.06.2005
(73) Proprietor: Moreno Molino, Braulio F., 18194 Cullar Vega (Granada) (ES)
(72) Inventor: Moreno Molino, Braulio F., 18194 Cullar Vega (Granada) (ES)
(74) Representative: Isern-Jara, Nuria

(56) References cited:
- EP-A- 1 217 075
- EP-A- 1 354 644
- DE-A1- 19 623 163
- DE-A1- 19 628 521

## Description

### FIELD OF THE INVENTION

This invention refers to a procedure for eliminating meat wastes, especially animal cadavers.

As expressed in the title, this is a method for conveniently destroying meat, particularly the meat of complete or partial bodies of animals of different species.

### HISTORY

The elimination of animal remains constitutes a classic problem that has tried to be solved in different ways: deposition in the open, deposition for ecological purposes, burying, direct or selective cremation, and others.

Specifically, cremation generally suffers from the fact the combustion is not perfect in time, highly contaminating and cancerigenic products are produced, the investment needed for cremating installations and the energetic cost are very high and, usually, a serious attack on the environment and unpleasantness for persons are caused.

None of the mentioned systems constitutes a solution to the problem presented, as a serious aggression on the environment is caused due to the attraction of vermin and other animals, production of nauseating materials, gases, smoke, soil and water contamination, offense to the sight of the rotting wastes, etc.

It is known from EP-A-1354644 a process for eliminating meat wastes by means of a tank in an aerobic or anaerobic manner. However, it has been found that the above process does not obtain a sufficiently satisfactory result as desired in comparison with the process of the present invention as disclosed below.

This document discloses the features of said Patent Application in the preamble of the claim 1.

It has become urgent and necessary to find a new procedure that permits the convenient elimination of meat wastes and animal cadavers without attacking the environment or generating unpleasant effects for persons. This has been achieved by the features of claim 1 and by putting into practice this invention, which proposes a new system to treat and destroy organic materials coming from the bodies of animals. Further embodiments of the invention are defined in the dependent claims.

The procedure to be described will find its most suitable raison d'etre in the event of epidemics and illnesses of stock cattle, where large quantities of meat materials or similar are produced.

### DESCRIPTION OF THE INVENTION

The new procedure consists in treating the materials intended to be eliminated by following a method based on hydrolization, that is, on the use of an aqueous method combined with the action of an active product that destroys the meat wastes and the action of degrading bacteria.

Hydrolization is justified by the high content of water in all living species (content that varies between 49% and 97%, according to the species), by the very limited presence of air and water aggressive agent emission and by the reduced number of handling operations of the materials to be eliminated and which could cause unpleasantness for the workers carrying out the procedure.

The means for operation is water, activated by a catalyst and maintained in a digester in which the bodies of the animals are deposited as the contents.

Anaerobic (without air) fermentation is caused in a liquid stage (aqueous) with the addition of an activator (preferably integral sodium alginate) that favors the liquation by hydrolization of the meat materials when operating as a catalyst.

The process in question is basically physical and/or biological and not chemical.

The digestor used in the practice of this invention can be of the conventional type, in the shape of a cylindrical or spherical tank with stiff walls, made of a material not prone to attacks by corrosive agents, for example, a high resistant synthetic plastic, and with complementary elements to be detailed later on.

The already mentioned activating product is an integral sodium alginate liquid dispersion concentrate obtained from 100% seaweed and gives a very basic character with high capacity for ionic exchange and a sealing effect as regards the acids. It is presented in the form a fibrous jelly with particles of up to 1 mm, brown in color and easily dispersed in water, above all in warm water. This product has a high content of soluble fibers, high quality proteins with a polyneral structure in which the alginic acid, oligosaccharides and a high proportion of bio-active iodine are represented. It interacts with the ammonium radicals and results in the so-called sponge effect (existence of non-flowing jelly), which is translated into a kidnapping and catalysation of the oligogases, which give rise to degrading bacteria for their transformation.

The product in question accelerates the rotting process of the meat bodies and materials. The soft and bony masses become liquefied within a period of one to three months according to their composition.

The digestion of the materials occurs in a liquid phase (fermentation with the production of high temperatures) and requires the addition of water (prior to adding the activating product) to consume the bacteria. The meat product must be covered with at least two thirds of water.

The animal proteins are degraded by the bacteria (especially the soluble materials, mesophilic phase at 35° - 60° C), by means of actinomycete fungi (especially the polymers, termophilic phase at 60°-70° C) and by enzymes (particularly the hard parts, mesophilic phase at 70°-40° C).

The remaining elements of the meat structures (nitrogen, phosphorous, calcium, etc.) appear in an organic and chelate form completely hygienic due to the high temperatures reached during the fermentation process.

## Claims

1. Procedure for eliminating meat wastes, especially animal cadavers, intended for non-aggressive destruction as regards the environment of whole and/or partial bodies of animals of different species, including the physical and biological treatment of the materials to be eliminated by means of a controlled anaerobic fermentation process in liquid phase, in a partially closed container, of the material to be eliminated, in a basically aqueous environment, with the cooperation of a bio-active product, the action of degrading bacteria and the application of heat, **characterized by** the fact that the bio-active product, operating as a cooperating catalyst during the treatment process, is integral sodium alginate in 100% concentrated liquid dispersion, with a very basic character and high ionic exchange capacity.

2. Procedure for eliminating meat wastes, especially animal cadavers, according to claim 1, **characterized by** the fact the treatment container is buried at approximately three quarters of its height with its upper part emerging in approximately a fourth part of its height, being upper mouths of the container accessible.

3. Procedure for eliminating meat wastes, especially animal cadavers, according to the above claims, **characterized by** the fact that initial loading of the treatment container is verified with the introduction of the materials to be transformed and the cooperating bio-active product, as well as with water up to a minimum height of three quarters of the height of the mass of the introduced materials.

4. Procedure for eliminating meat wastes, especially animal cadavers, according to claim 1, **characterized by** the fact that degradation, by liquefaction, of the soft and soluble proteinic materials is verified by bacteria in the mesophilic phase at temperatures between 35° and 60°C.

5. Procedure for eliminating meat wastes, especially animal cadavers, according to claim 1, **characterized by** the fact the degradation by liquefaction of the soluble polymeric materials is verified by actinomycete fungi in the thermophilic phase at temperatures between 60° and 70°C.

6. Procedure for eliminating meat wastes, especially animal cadavers, according to claim 1, **characterized by** the fact the degradation by liquefaction of the materials of a greater consistency and hardness is verified by enzymes in the mesophilic phase at temperatures between 70° and 40°C.

7. Procedure for eliminating meat wastes, especially animal cadavers, according to claim 1, **characterized by** the fact the result of treating the organic materials is the obtaining of a bio-degraded material in a liquid-paste state, able to be extracted by means of a suction pump suitable for dense liquids and to be incorporated in products such as fertilizers and similar that act as final elimination vehicles, without generating harmful or offensive effects for the environment.

## Patentansprüche

1. Verfahren zur Entsorgung von Fleischabfällen, insbesondere von Tierkadavern, mit dem ganze und/oder Teile von Tierkörpern verschiedener Spezies einer umweltschonenden Vernichtung zugeführt werden können, einschließlich physikalischer und biologischer Behandlung der zu entsorgenden Materialien mittels eines kontrollierten anaeroben Gärungsprozesses in flüssiger Phase in einem teilweise geschlossenen Behälter in einer im Wesentlichen wässrigen Umgebung unter Mitwirkung eines bioaktiven Produktes, der Wirkung zersetzender Bakterien und Wärmeanwendung, **dadurch gekennzeichnet, dass** das bioaktive Produkt, das im Behandlungsprozess die Funktion eines kooperierenden Katalysators hat, ein integrales Natriumalginat in 100% konzentrierter Flüssigdispersion mit einem sehr basischen Charakter und hoher Ionenaustauschfähigkeit ist.

2. Verfahren zur Entsorgung von Fleischabfällen, insbesondere von Tierkadavern, nach Anspruch 1 und 2, **dadurch gekennzeichnet dass** der Behandlungscontainer zu etwa drei Viertel seiner Höhe eingegraben wird und der obere Teil etwa zu einem Viertel hervorragt, wobei obere Öffnungen des Containers zugänglich sind.

3. Verfahren zur Entsorgung von Fleischabfällen, insbesondere von Tierkadavern, nach einem der vorhergehenden Ansprüche, **dadurch gekennzeichnet, dass** das anfängliche Beladen des Behandlungscontainers durch das Einbringen des zu verarbeitenden Materials und des kooperierenden bioaktiven Produktes, sowie das Einfüllen von Wasser bis zu mindestens drei Viertel der Höhe der eingebrachten Materialmasse erfolgt.

4. Verfahren zur Entsorgung von Fleischabfällen, insbesondere von Tierkadavern, nach Anspruch 1, **dadurch gekennzeichnet, dass** der Abbau des weichen und löslichen Proteinmaterials durch Verflüssigung mittels Bakterien in der mesophilen Phase bei einer Temperatur zwischen 35º und 60º C erfolgt.

5. Verfahren zur Entsorgung von Fleischabfällen, insbesondere von Tierkadavern, nach Anspruch 1, **dadurch gekennzeichnet, dass** der Abbau des löslichen Polymermaterials durch Verflüssigung mittels Actinomyceten in der thermophilen Phase bei einer Temperatur zwischen 60º und 70º C erfolgt.

6. Verfahren zur Entsorgung von Fleischabfällen, insbesondere von Tierkadavern, nach Anspruch 1, **dadurch gekennzeichnet, dass** der Abbau von Materialien größerer Konsistenz und Härte durch Verflüssigung mittels Enzymen in der mesophilen Phase bei einer Temperatur zwischen 70º und 40º C erfolgt.

7. Verfahren zur Entsorgung von Fleischabfällen, insbesondere von Tierkadavern, nach Anspruch 1, **dadurch gekennzeichnet, dass** man als Ergebnis der Behandlung des organischen Materials ein biologisch abbaubares Material in einem flüssig-breiigen Zustand erhält, das mit der Hilfe einer für dichte Flüssigkeiten geeigneten Saugpumpe entnommen und in Produkte wie Dünger und Ähnliches eingebracht werden kann, welche als endgültige Entsorgungsträger agieren ohne eine die Umwelt beeinträchtigende oder schädigende Auswirkung zu haben.

## Revendications

1. Procédé d'élimination des déchets de la viande, notamment des cadavres d'animaux, destiné à la destruction non agressive pour l'environnement de corps entiers ou partiels d'animaux de différentes espèces, ledit procédé incluant le traitement physique et biologique, sous forme d'une fermentation anaérobie contrôlée en phase liquide, dans un récipient partiellement fermé, des matières à éliminer, dans un milieu essentiellement aqueux, à l'aide d'un produit bioactif, de l'action de bactéries dégradantes et de l'application de chaleur, **caractérisé en ce que** ledit produit bioactif fonctionne comme un catalyseur qui contribue au procédé de traitement, est un alginate sodique en dispersion liquide concentrée à 100 % de nature très basique et possédant une capacité d'échange ionique élevée.

2. Procédé d'élimination des déchets de la viande, notamment des cadavres d'animaux, selon la revendication 1 et 2, **caractérisé en ce que** les trois quarts environ de la hauteur du récipient de traitement sont enterrés et qu'un quart environ de la partie supérieure du récipient reste en surface, les ouvertures supérieures du récipient pouvant être ouvertes.

3. Procédé d'élimination des déchets de la viande, notamment des cadavres d'animaux, selon les revendications précédentes, **caractérisé en que** le chargement initial du récipient de traitement est vérifié avec l'introduction des matières à transformer et du produit bioactif coopérant, ainsi que de l'eau jusqu'aux trois quarts, au moins, de la hauteur de la masse des matières introduites.

4. Procédé d'élimination des déchets de la viande, notamment des cadavres d'animaux, selon la revendication 1, **caractérisé en ce que** la dégradation, par liquidation, des matières protéiques molles et solubles est vérifiée au moyen de bactéries en phase mésophile à des températures de 35º à 60 ºC.

5. Procédé d'élimination des déchets de la viande, notamment des cadavres d'animaux, selon la revendication 1, **caractérisé en ce que** la dégradation, par liquidation, des matières polymériques solubles est vérifiée au moyen de champignons actinomycètes en phase thermophile à des températures de 60º à 70 ºC.

6. Procédé d'élimination des déchets de la viande, notamment des cadavres d'animaux, selon la revendication 1, **caractérisé en ce que** la dégradation, par liquidation, des matières de plus grande consistance et dureté est vérifiée au moyen d'enzymes en phase mésophile à des températures de 70º à 40 ºC.

7. Procédé d'élimination des déchets de la viande, notamment des cadavres d'animaux, selon la revendication 1, **caractérisé en ce que** le traitement des matières organiques a pour résultat d'obtenir une matière biodégradée à l'état liquide-pâteux, susceptible d'être extraite au moyen d'une pompe d'aspiration appropriée pour les liquides denses et d'être incorporée, sans la production d'effets nuisibles ou offensifs pour l'environnement, à des produits tels que des fertilisants et similaires, agissant comme des véhicules d'élimination finale.
